# EUROPEAN PATENT SPECIFICATION

(11) **EP 1 605 937 B1**
(45) Date of publication and mention of the grant of the patent: **15.12.2010**
(21) Application number: 04719972.4
(22) Date of filing: 12.03.2004
(51) Int. Cl.: A61K 31/427, A61P 35/00

(54) **TREATMENT OF PROLIFERATIVE DISEASES WITH EPOTHILONE DERIVATIVES AND RADIATION**
BEHANDLUNG PROLIFERATIVER ERKRANKUNGEN MIT EPOTHILON-DERIVATEN UND BESTRAHLUNG
TRAITEMENT DE MALADIES PROLIFERATIVES AU MOYEN DE DERIVES D'EPOTHILONE ET D'UNE EXPOSITION A DES RAYONNEMENTS

(30) Priority: 14.03.2003 GB 0305928
(43) Date of publication of application: 21.12.2005
(73) Proprietor: Novartis AG, 4056 Basel (CH); Novartis Pharma GmbH, 1230 Wien (AT)
(72) Inventor: PRUSCHY, Martin, CH-8802 Kilchberg (CH)
(74) Representative: de Weerd, Petrus G.W.
(86) International application number: PCT/EP2004/002610
(87) International publication number: WO 2004/080458

(56) References cited:
- WO-A-99/02514
- WO-A-02/058700
- PATENT ABSTRACTS OF JAPAN vol. 2002, no. 02, 2 April 2002 (2002-04-02) & JP 2001 288097 A (PG-TXL CO LP), 16 October 2001 (2001-10-16)
- KIM JAE-CHUL ET AL: "Potential radiation-sensitizing effect of semisynthetic epothilone B in human lung cancer cells." RADIOTHERAPY & ONCOLOGY, vol. 68, no. 3, September 2003 (2003-09), pages 305-313, XP002283036 ISSN: 0167-8140
- HOFSTETTER BARBARA ET AL: "Patupilone acts as radiosensitizing agent in multidrug-resistant cancer cells in vitro and in vivo." CLINICAL CANCER RESEARCH : AN OFFICIAL JOURNAL OF THE AMERICAN ASSOCIATION FOR CANCER RESEARCH 15 FEB 2005 LNKD- PUBMED:15746064, vol. 11, no. 4, 15 February 2005 (2005-02-15), pages 1588-1596, ISSN: 1078-0432

## Description

This invention relates to organic compounds, in particular to pharmaceutical compositions for use in combination with ionizing radiation for the delay of progression or treatment of a proliferative disease, especially a solid tumor disease.

We have now found that certain Epothilone derivatives are effective when used in combination with ionizing radiation for the delay of progression or treatment of a proliferative disease, especially a solid tumor disease:

Accordingly the invention provides an epothilone derivative of formula I 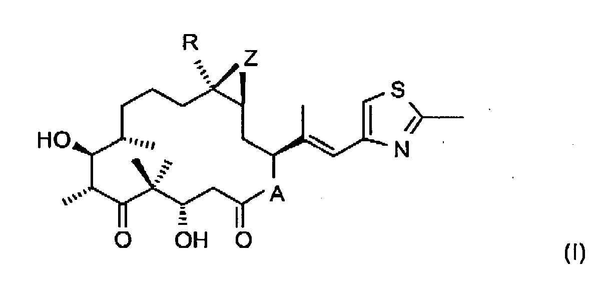 in which compound A represents O, R is methyl, and Z is O which is in free form or in the form of a pharmaceutically acceptable salt and optionally at least one pharmaceutically acceptable carrier for use in combination with ionizing radiation, in the delay of progression or treatment of a proliferative disease, especially a solid tumor disease.

A compound of formula I wherein A represents O, R is hydrogen and Z is O is known as epothilone A; a compound of formula I wherein A represents O, R is methyl and Z is O is known as epothilone B: a compound of formula I wherein A represents O, R is hydrogen and Z is a bond is known as epothilone C; a compound of formula I wherein A represents O, R is methyl and Z is a bond is known as epothilone D.

Further the invention provides the use of epothilone (or pharmaceutically acceptable salt thereof) for the preparation of a medicament for use in combination with ionizing radiation in the treatment of a proliferative disease.

In a further aspect the invention provides use of epothilone B (or pharmaceutically acceptable salt thereof) in combination with ionizing radiation for the treatment of a proliferative disease, especially a solid tumor.

In yet further aspect the invention provides an epothilone B (or pharmaceutically acceptable salt thereof) as active ingredient for use in combination with ionizing radiation for the treatment of a proliferative disease, especially a solid tumor.

Above and elsewhere in the present description the following terms have the meanings given below:
The term "delay of progression" as used herein means administration of the combination to patients being in an early phase of the proliferative disease to be treated.
The term "solid tumor disease" as used herein comprises, but is not restricted to glioma, thyroid cancer, breast cancer, ovarian cancer, cancer of the colon and generally the GI tract, cervix cancer, lung cancer, in particular small-cell lung cancer, and non-small-cell lung cancer, head and neck cancer, bladder cancer, cancer of the prostate or Kaposi's sarcoma. In one preferred embodiment of the invention, the tumor disease to be treated is glioma, cancer of the prostate or thyroid cancer. The present combination inhibits the growth of solid tumors, but also liquid tumors. Furthermore, depending on the tumor type and the particular combination used, a decrease of the tumor volume can be obtained. The combinations disclosed herein are also suited to prevent the metastatic spread of tumors and the growth or development of micrometastases.

Combination refers to administration of an amount of epothilone derivative of formula I in combination with administration of an amount of ionizing radiation such that there is a synergistic effect which would not be obtained if an epothilone derivative of formula I is administered without separate, simultaneous or sequential administration of ionizing radiation. Wherein administration of ionizing radiation can be continuous, sequential or sporadic. Or an effect which would not be obtained if there is administered ionizing radiation without the separate, simultaneous or sequential administration of an Epothilone derivative of formula I, wherein administration can be continuous, sequential or sporadic Preferably combination refers to administration of an amount of epothilone derivative of formula I in combination with administration of an amount of ionizing radiation such that there is a synergistic antiproliferative effect and/ or a clonogenic cell killing effect that would not be obtained if
a) The epothilone derivative of formula I is administered without prior, simultaneous or subsequent administration of ionizing radiation. Wherein administration can be continuous, sequential or sporadic;
b) There is administration of ionizing radiation without the prior, simultaneous or subsequent administration of an epothilone derivative of formula I. Where in administration can be continuous, sequential or sporadic.

The term "ionising radiation" referred to above and hereinafter means ionising radiation that occurs as either electromagnetic rays (such as X-rays and gamma rays) or particles (such as alpha and beta particles). Ionising radiation is provided in, but not limited to, radiation therapy and is known in the art (Hellman, Principles of Radiation Therapy, Cancer, in Principles and Practice of Oncology, 248-275 (Devita et al., ed., 4th Ed., V1, 1993).

Epothilone derivatives of formula I wherein A represents O, R is methyl and Z is O, and methods for the preparation of such epothilone derivative are in particular generically and specifically disclosed in the patents and patent applications WO 93/10121, US 6,194,181, WO 98/25929, WO 98/08849, WO 99/43653, WO 98/22461 and WO 00/31247. Comprised are likewise the corresponding stereoisomers as well as the corresponding crystal modifications, e.g. solvates and polymorphs, which are disclosed therein.

The transformation of epothilone B to the corresponding lactam is disclosed in Scheme 21 (page 31, 32) and Example 3 of WO 99/02514 (pages 48 - 50). The transformation of a compound of formula I which is different from epothilone B into the corresponding lactam can be accomplished analogously. Corresponding epothilone derivatives of formula I wherein RN is lower alkyl can be prepared by methods known in the art such as a reductive alkylation reaction starting from the epothilone derivative wherein RN is hydrogen.

Epothilone B, can be administered as part of pharmaceutical compositions which are disclosed in WO 99/39694.

A combination which comprises (a) an epothilone derivative of formula I in which compound A represents O, R is methyl and Z is O which may be present in free form or in the form of a pharmaceutically acceptable salt and optionally at least one pharmaceutically acceptable carrier and (b) ionizing radiation, will be referred to hereinafter as a COMBINATION OF THE INVENTION.

The nature of proliferative diseases like solid tumor diseases is multifactorial. Under certain circumstances, drugs with different mechanisms of action may be combined. However, just considering any combination of drugs having different mode of action does not necessarily lead to combinations with advantageous effects.

In the combination of the invention, Epothilone B and pharmaceutically acceptable salts are preferably used in the form of pharmaceutical preparations that contain the relevant therapeutically effective amount of active ingredient optionally together with or in admixture with inorganic or organic, solid or liquid, pharmaceutically acceptable carriers which are suitable for administration.

In a preferred embodiment, each patient receives doses of ionizing radiation, whereas the epothilone derivative of formula I is administered once weekly i.v. for three weeks, followed by one week off. Each four week interval will be considered one cycle. Day 1 of each cycle is defined as the day of administration of epothilone derivative of formula I and ionizing radiation. The efficacy of the treatment can be determined in these studies, e.g., after 18 or 24 weeks by radiologic evaluation of the tumors every 6 weeks.

In an alternative embodiment, the ionizing radiation is given as a pre-treatment, i.e. before the treatment with the COMBINATION OF THE INVENTION is started; the ionizing radiation alone is administered to the patient for a defined period of time, e.g. daily administration of the ionizing radiation alone for two or three days or weeks.

In another preferred embodiment, the epothilone derivative of formula I is administered once weekly i.v. for three weeks, followed by one week off. Each four week interval will be considered one cycle. The efficacy of the treatment can be determined in these studies, e.g., after 18 or 24 weeks by radiologic evaluation of the tumors every 6 weeks.

The Epothilone B pharmaceutical compositions may be, for example, compositions for enteral, such as oral, rectal, aerosol inhalation or nasal administration, compositions for parenteral, such as intravenous or subcutaneous administration, or compositions for transdermal administration (e.g. passive or iontophoretic), or compositions for topical administration,

Preferably, the Epothilone B pharmaceutical compositions are adapted to oral administration.

The pharmaceutical compositions according to the invention can be prepared in a manner known per se and are those suitable for enteral, such as oral or rectal, and parenteral administration to mammals (warm-blooded animals), including man, comprising a therapeutically effective amount of at least one pharmacologically active combination partner alone or in combination with one or more pharmaceutically acceptable carries, especially suitable for enteral or parenteral application.

The novel pharmaceutical composition contain, for example, from about 10 % to about 100 %, preferably from about 20 % to about 60 %, of the active ingredients. Pharmaceutical preparations for the combination therapy for enteral or parenteral administration are, for example, those in unit dosage forms, such as sugar-coated tablets, tablets, capsules or suppositories, and furthermore ampoules. If not indicated otherwise, these are prepared in a manner known per se, for example by means of conventional mixing, granulating, sugar-coating, dissolving or lyophilizing processes. It will be appreciated that the unit content of a combination partner contained in an individual dose of each dosage form need not in itself constitute an effective amount since the necessary effective amount can be reached by administration of a plurality of dosage units.

In preparing the compositions for oral dosage form, any of the usual pharmaceutical media may be employed, such as, for example, water, glycols, oils, alcohols, flavouring agents, preservatives, colouring agents; or carriers such as starches, sugars, microcrystalline cellulose, diluents, granulating agents, lubricants, binders, disintegrating agents and the like in the case of oral solid preparations such as, for example, powders, capsules and tablets, with the solid oral preparations being preferred over the liquid preparations. Because of their ease of administration, tablets and capsules represent the most advantageous oral dosage unit form in which case solid pharmaceutical carriers are obviously employed.

In particular, a therapeutically effective amount of each combination partner of the COMBINATION OF THE INVENTION may be administered simultaneously or sequentially and in any order, and the components may be administered separately or as a fixed combination. For example, the method of delay of progression or treatment of a proliferative disease according to the invention may comprise (i) administration of the first combination partner and (ii) administration of the second combination partner, wherein administration of a combination partner may be simultaneous or sequential in any order, in jointly therapeutically effective amounts, preferably in synergistically effective amounts, e.g. in daily or weekly dosages corresponding to the amounts described herein. The individual combination partners of the COMBINATION OF THE INVENTION can be administered separately at different times during the course of therapy or concurrently. Furthermore, the term administering also encompasses the use of a pro-drug of an epothilone derivative of formula I that converts *in vivo* to the combination partner as such. The instant invention is therefore to be understood as embracing all such regimes of simultaneous or alternating treatment and the term "administering" is to be interpreted accordingly.

The dosage of ionizing radiation and an epothilone derivative of formula I in relation to each other is preferably in a ratio that is synergistic.

If the warm-blooded animal is a human, the dosage of a compound of formula I is preferably in the range of about 0.25 to 75, preferably 0.5 to 50, e.g. 2.5, mg/m² once weekly for two to four, e.g. three, weeks, followed by 6 to 8 days off in the case of an adult patient. In one embodiment of the invention, epothilone B is administered in accordance with the treatment schedule described in US 6,302,838.

The particular mode of administration and the dosage of a compound of formula I may be selected by the attending physician taking into account the particulars of the patient, especially age, weight, life style, activity level, etc.

The dosage of an epothilone of formula I may depend on various factors, such as effectiveness and duration of action of the active ingredient, mode of administration, effectiveness and duration of action of the ionizing radiation and/or sex, age, weight and individual condition of the subject to be treated.

The dosage of ionizing radiation may depend on various factors, such as effectiveness and duration of action of the ionizing radiation, mode of administration, location of administration, effectiveness and duration of action of the epothilone derivative of formula I and/or sex, age, weight and individual condition of the subject to be treated. The dosage of ionizing radiation is generally defined in terms of radiation absorbed dose, time and fraction, and must be carefully defined by the attending physician.

Salt-forming groups in a compound of formula I are groups or radicals having basic or acidic properties. Compounds having at least one basic group or at least one basic radical, for example a free amino group, a pyrazinyl radical or a pyridyl radical, may form acid addition salts, for example with inorganic acids, such as hydrochloric acid, sulfuric acid or a phosphoric acid, or with suitable organic carboxylic or sulfonic acids, for example aliphatic mono- or di-carboxylic acids, such as trifluoroacetic acid, acetic acid, propionic acid, glycolic acid, succinic acid, maleic acid, fumaric acid, hydroxymaleic acid, malic acid, tartaric acid, citric acid or oxalic acid, or amino acids such as arginine or lysine, aromatic carboxylic acids, such as benzoic acid, 2-phenoxy-benzoic acid, 2-acetoxy-benzoic acid, salicylic acid, 4-aminosalicylic acid, aromatic-aliphatic carboxylic acids, such as mandelic acid or cinnamic acid, heteroaromatic carboxylic acids, such as nicotinic acid or isonicotinic acid, aliphatic sulfonic acids, such as methane-, ethane- or 2-hydroxyethane-sulfonic acid, or aromatic sulfonic acids, for example benzene-, p-toluene- or naphthalene-2-sulfonic acid. When several basic groups are present mono- or poly-acid addition salts may be formed.

For the purposes of isolation or purification, as well as in the case of compounds that are used further as intermediates, it is also possible to use pharmaceutically unacceptable salts.

Only pharmaceutically acceptable, non-toxic salts are used for therapeutic purposes, however, and those salts are therefore preferred.

In the combination of the invention comprises epothilone B and ionizing radiation.

Furthermore, the present invention pertains to the use of a COMBINATION OF THE INVENTION for the delay of progression or treatment of a proliferative disease and for the preparation of a medicament for the delay of progression or treatment of a proliferative disease.

In one embodiment of the invention, an antidiarrheal agent is administered together with the COMBINATION OF THE INVENTION in order to prevent, control or eliminate diarrhoea that is sometimes associated with the administration of epothilones, especially epothilone B. Thus, the present invention finds utility in preventing or controlling diarrhoea associated with administering an epothilone derivative of formula I, which comprises administering an effective amount of an antidiarrhea agent to the patient receiving treatment with the COMBINATION OF THE INVENTION. Antidiarrheal agents and protocols for their administration are known to those skilled in the art. Antidiarrheal agents suitable for use in the inventive methods and compositions include, but are not limited to, natural opiods, such as tincture of opium, paregoric, and codeine, synthetic opioids, such as diphenoxylate, difenoxin and loperamide, bismuth subsalicylate, octreotide (e.g. available as SANDO-STATINTM), motilin antagonists and traditional antidiarrheal remedies, such as kaolin, pectin, berberine and muscarinic agents.

The following example is intended to illustrate the invention and are not to be construed as being limitations thereon.

### Example 1

Tumor cell proliferation was assessed by the colorimetric MTT-like alamarBlue assay that is based on detection of metabolite activity. To determine clonogenic survival the number of singular cells plated was adjusted to obtain about 100 colonies per dish with a given treatment. After 24 h exposure to the different drugs cells were irradiated and then allowed to grow for 8 to 10 days before fixation in methanol/acetic acid (75%/25%) and staining with crystal violet. Only colonies with more than 50 cells/colony were counted. The plating efficiency (PE) of untreated cells was determined and calculated as PE (%) = (scored colonies/number of plated cells)x100. The surviving fraction (SF) with a given treatment was determined by SF = (scored colonies) / (number of plated cells x PE/100). Clonogenic assays were performed at least twice and absence of error bars is due to minimal standard deviations. Irradiation of cell cultures was carried out at RT in tissue culture dishes (100x100mm) or in 96-well plates using a Pantak Therapax 3,300 kV X-ray unit at 0.7 Gy/min. Dosimetry was controlled with a Vigilant-dosimeter.

Initial proliferation assays were performed to determine the dose range of epothilone B to be applied for combined treatment with ionizing radiation. A clear antiproliferative dose response over 72 hours against the two cell lines (E1A/ras transformed p53-/- MEF; human colon adenocarcinoma cell line SW480) was observed in a subnanomolar and low nanomolar range of epothilone B. The human SW480 cells showed enhanced sensitivity to epothilone B than the genetically defined oncogene-transformed MEFs (Appendix epothilone B-1). Combined treatment with epothilone B and ionizing radiation (5Gy) revealed an at least additive antiproliferative effect against these two cell lines (Appendix epothilone B-2, shown with a representative concentration of epothilone B). For this combined treatment modality cells were pretreated for 24h with epothilone B prior to irradiation.

Based on these results, clonogenic survival assays were performed with epothilone B in combination with ionizing radiation. The clonogenic assay is based on the outgrowth of compact clones from singular cells that are seeded at low density in a petri dish. Clonal outgrowth under the different treatment conditions can be quantified and compared.

Appendix epothilone B-3 summarizes clonogenic survival performed with SW480 and E1A/ras-trasnsormed MEFs upon treatment with ionizing radiation and epothilone B alone and in combination. Similar to the proliferation assay SW480 cells were more sensitive to epothilone B than the MEFs and combined treatment showed again an at least additive effect in both cell lines (dose range for epothilone B are different for SW480 and MEFs as illustrated in the respective graphs). Based on these results efficacy of combined treatment should be tested in vivo using tumor allograft/xenograft models.

Based on the interesting profile of epothilone B to be antiproliferative also in (some) Paclitaxel-refractory tumor cell lines we compared the effect of epothilone B and Paclitaxel in combination with ionizing radiation against these two cell lines. The human SlU480 colon cancer cell line proofed to be refractory to Paclitaxel (doses up to 500 nM, Appendix 4a).

Though Paclitaxel and epothilone B reduced the proliferative activity alone and in combination with ionizing radiation in the murine fibrosarcoma cell line to a comparable extent (in a low nanomolar range, Appendix 4b).

We compared the effect of combined treatment with Epothilone B/IR and PaclitaxeI/IR also in the clonogenic cell survival assay. Both cell lines showed an at least additive effect to combined treatment with epothilone B/IR and Paclitaxel /IR respectively, but only at very high concentrations of Paclitaxel in the Paclitaxel-resistant cell line SW480 (Appendix epothilone B-5).

Paclitaxel-resistance is often due to MDR-P-glycoprotein-overexpression in tumor cells, inhibitable by the MDR-reversal agent verapamil. Therefore proliferation experiments were performed with the different treatment modalities in SW480 cells pretreated with verapamil. Low doses of verapamil (5 µg/ml, added 30 min prior to Paclitaxel-treatment) resensitized SW480 cells to low doses of Paclitaxel alone and to a combined treatment modality with ionizing radiation indicating that MDR-P-glycoprotein-overexpression is responsible for the Paclitaxel-refractory effect in this cell line (Appendix epothilone B-6). Verapamil did not have an antiproliferative effect by itself at this concentration and only slightly affected the response to epothilone B (not shown). We are currently probing the level of MDR-P-glycoprotein in a direct way in SW480 cells by immunoblotting.

Overall these results show that both epothilone B and paclitaxel have an at least additive antiproliferative and clonogenic cell killing effect in combination with ionizing radiation, epothilone B retains full activity alone and in combination with ionizing radiation in the Paclitaxel-resistant human colon adenocarcinoma cell line SW480. Thus Epothilone might be a promising alternative in paclitaxel resistant tumors (e.g. colorectal tumors) for a combined treatment regimen using IR and microtubule inhibitors. Experimentally the next steps will involve in vivo testing of epothilone B/IR with allograft/xenograft mouse tumor models.

## Claims

1. An epothilone derivative of formula I 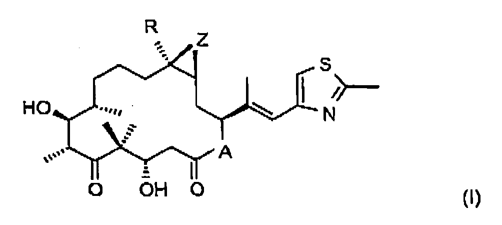 in which compound A represents O, R is methyl and Z is O, which is in free form or in the form of a pharmaceutically acceptable salt, for use in combination with ionizing radiation, in the delay of progression or in the treatment of a proliferative disease.

2. The use of (a) an epothilone derivative of formula I 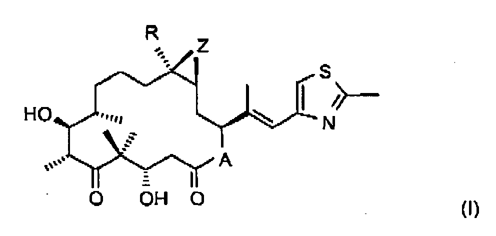 in which compound A represents O, R Is methyl and Z is O, which is in free form or in the form of a pharmaceutically acceptable salt, for the preparation of a medicament for use in combination with
(b) ionizing radiation, for the delay of progression or treatment of a proliferative disease.

3. A use according to claim 2 which comprises administering a quantity which is jointly therapeutically effective against a proliferative disease of a compound of formula I and at least one pharmaceutically acceptable carrier for use in combination with ionizing radiation.

4. A use according to claims 2 or 3 wherein the proliferative disease is a solid tumor.

## Patentansprüche

1. Ein Epothilonderivat der Formel I 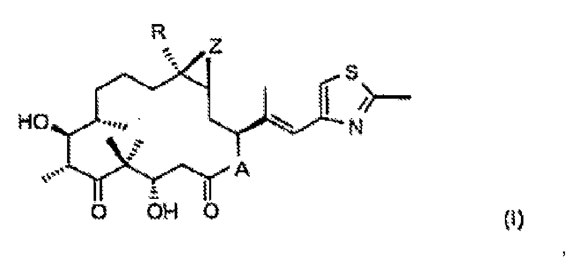 bei dem Verbindung A für O steht, R Methyl ist und Z gleich O ist, das in der freien Form oder in der Form eines pharmazeutisch akzeptierbaren Salzes vorliegt, zur Verwendung in Kombination mit ionisierender Strahlung, zur Verzögerung des Fortschreitens oder zur Behandlung einer proliferativen Erkrankung.

2. Die Verwendung von (a) einem Epothilonderivat der Formel I 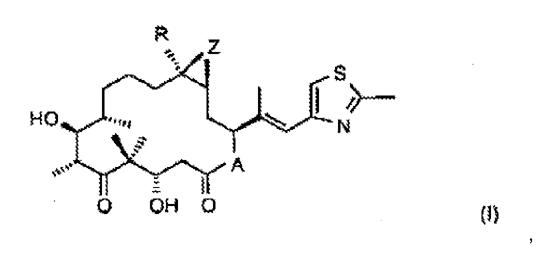 bei dem Verbindung A für O steht, R Methyl ist und Z gleich O ist, das in der freien Form oder in der Form eines pharmazeutisch akzeptierbaren Salzes vorliegt, zur Herstellung eines Arzneimittels zur Verwendung in Kombination mit
(b) ionisierender Strahlung, zur Verzögerung des Fortschreitens oder Behandlung einer proliferativen Erkrankung.

3. Eine Verwendung gemäß Anspruch 2, welche die Verabreichung einer Menge, die insgesamt therapeutisch wirksam gegen eine proliferative Erkrankung ist, einer Verbindung der Formel I und mindestens einem pharmazeutisch akzeptablen Trägerstoff umfasst, zur Verwendung in Kombination mit ionisierender Strahlung.

4. Eine Verwendung gemäß den Ansprüchen 2 oder 3, bei der die proliferative Erkrankung ein solider Tumor ist.

## Revendications

1. Dérivé d'épothilone de formule I 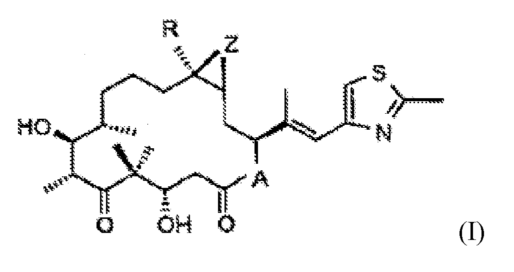 dans laquelle A représente O, R est un groupe méthyle et Z représente O, qui est sous forme libre ou sous forme d'un sel pharmaceutiquement acceptable, à utiliser en combinaison avec un rayonnement ionisant, pour retarder la progression ou pour le traitement d'une maladie proliférative.

2. Utilisation (a) d'un dérivé d'épothilone de formule I 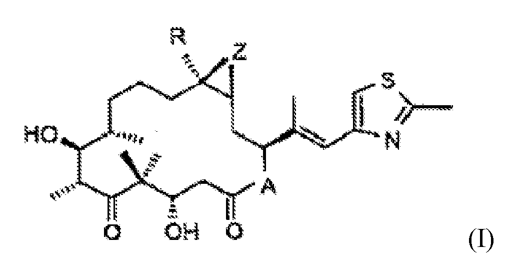 dans laquelle A représente O, R est un groupe méthyle et Z représente O, qui est sous forme libre ou sous forme d'un sel pharmaceutiquement acceptable, pour la préparation d'un médicament à utiliser en combinaison avec
(b) un rayonnement ionisant, pour retarder la progression ou pour le traitement d'une maladie proliférative.

3. Utilisation selon la revendication 2, comprenant l'administration d'une quantité, qui est conjointement efficace sur le plan thérapeutique contre une maladie proliférative, d'un composé de formule I et d'au moins un véhicule pharmaceutiquement acceptable à utiliser en combinaison avec un rayonnement ionisant.

4. Utilisation selon la revendication 2 ou 3, où la maladie proliférative est une tumeur solide.
